# EUROPEAN PATENT APPLICATION

(11) **EP 4 368 616 A1**
(43) Date of publication of application: **15.05.2024**
(21) Application number: 22207250.6
(22) Date of filing: 14.11.2022
(51) Int. Cl.: C07D 307/48, C08G 63/90, C08L 67/02

(54) **A METHOD FOR PREPARING A PURIFIED CYCLIC COMPOUND COMPOSITION**

(71) Applicant: Sulzer Management AG, 8401 Winterthur (CH)
(72) Inventor: The designation of the inventor has not yet been filed
(74) Representative: Henkel & Partner mbB

(57) **Abstract**

A method for preparing a purified cyclic compound composition comprises the steps of:
i) providing in a reactor a reaction mixture containing at least one cyclizable linear compound and reacting the at least one cyclizable linear compound in a ring-closing reaction so as to obtain a crude composition containing at least one cyclic compound,
ii) feeding at least a portion of the crude composition obtained in step i) to a precipitator, in which the crude composition is cooled to a precipitation temperature being below the solubility temperature of at least one of the at least one cyclic compound contained in the crude composition for precipitating at least a portion thereof so as to obtain precipitated solid cyclic compound and unprecipitated liquid,
iii) separating at least a portion of the precipitated solid cyclic compound obtained in step ii) from the unprecipitated liquid so as to obtain a solid cyclic compound fraction and unprecipitated liquid fraction, and
iv) washing the solid cyclic compound fraction obtained in step iii) with a washing solvent having a temperature of above the precipitation temperature used in step ii) so as to obtain a washing solvent fraction and as purified cyclic compound composition a washed solid cyclic compound fraction and.

## Description

The present invention relates to a method for preparing a purified cyclic compound composition. Furthermore, the present invention relates to a purified cyclic compound composition being obtainable with the method, to the use of the purified cyclic compound composition and to a plant for preparing the purified cyclic compound composition.

Cyclic compounds, such as those comprising furan units, are important compounds, which are for instance used in the preparation of polyesters. Polyesters are an important class of commercial polymers with useful physical and mechanical properties and numerous applications. For instance, polyesters, such as polyethylene terephthalate (PET), polybutylene terephthalate (PBT) and polyacrylates, are commercially used as fibres, coatings, films or in composites. However, most of the industrial polyesters, such as in particular PET, PBT and polyacrylates, are produced from monomers, which are derived from petrochemical raw materials. On account of several reasons, such as in particular environmental friendliness, but also due to limited oil reserves, fluctuations of oil price and political instability in some production areas, there is growing interest for biobased polyesters produced from renewable raw materials.

Examples for biobased polyesters produced from renewable raw materials are polylactic acid (PLA) and polyhydroxyalkanoates (PHA). PHA is a linear polyester, which may be produced by microbial fermentation from sugars or lipids, and PLA, also a linear polyester, may be prepared by polycondensation of lactic acid or by a ring-opening polymerization of lactide, which is the cyclic diester of lactic acid. While PHA has not been widely industrialized due to limitations in production yields and downstream processing, PLA has a wide range of applications, because the technological and physiochemical properties of homo- and copolymers of lactic acid come quite close to the properties of those polymers derived from fossil-based resources. For instance, polylactic acid is used in the biomedical field in chirurgical implants, in films, such as e.g. in packagings, in fibers, such as e.g. for garments, in hygienic articles, in carpets and in disposable plastic products, such as e.g. disposable cutlery or containers. In addition, polylactic acid has found wide application in composite materials, such as in fiber-reinforced plastics. Other commercially used aliphatic biobased polyesters are polybutylene succinate (PBS) and polymers based on sebacic or adipic acids.

However, since aliphatic polyesters, such as PLA, are not suitable for replacing petrochemical-based aromatic polyesters in applications, such as higher temperature extrusion or molding or the production of bottles, biobased aromatic polyesters are of particular interest. Suitable biobased aromatic polyesters are those comprising furan units, such as furan-2,5-dicarboxylic acid (FDCA), 5-(hydroxymethyl)furan-2-carboxylic acid (HMFA) or 2,5-bis(hydroxyl methyl)furan (BHMF). On account of the chemical similarity of the furan ring to the phenyl ring, aromatic polyesters comprising furan units have similar properties as phenyl-based polymers, such as PET, and are thus are suitable to replace phenyl-based polymers.

Aromatic polyesters comprising furan units and methods for producing these are described for instance in WO 2014/139602 A1. More specifically, a cyclic polyester oligomer comprising furan units is reacted in the presence of a catalyst in a ring-opening polymerization step to a polyester polymer having furan units. This production method requires a comparable low reaction time and advantageously does not produce thermal degraded polymer by-products and also does not form high quantities of volatile side products, such as water or alcohol, which obviates the need of complex and costly high-capacity devolatilization systems for removing such volatile side products. However, this method requires as starting material a cyclic monomer comprising a furan unit or cyclic polyester oligomer comprising furan units in high purity of more than 99% by weight. Cyclic monomer comprising a furan unit and cyclic polyester oligomers comprising furan units may be produced, for instance, by reacting a linear monomer or oligomer in a ring-closing reaction to a respective cyclic compound, such as described for example in WO 2014/139603 A1. A particular suitable method for producing such aromatic cyclic compounds is the cyclodepolymerization of oligomers comprising furan units, which may be advantageously operated as distillation-assisted cyclodepolymerization, in which during the reaction byproducts and solvent are evaporated. However, the purity of the cyclic compounds obtained by these methods is usually less than 95%, because the purity of the cyclic compounds is limited by the extent of reaction, which is controlled by the thermodynamic equilibrium between the linear and the cyclic compounds. This also leads to a comparable long reaction time of up to 9 hours and high dilution of the cyclic compounds in the reaction mixture. Therefore, the cyclic compounds comprising furan unit produced in these methods need to be purified, for instance by silica adsorption, column chromatography and/or distillation, in order to have the required high purity enabling their use in a ring opening polymerization. However, such a purification drastically reduces the overall yield of the furan unit comprising cyclic compounds, such as to below 60%.

In view of this, the object underlying the present invention is to provide a simple and fast method for preparing a purified cyclic compound composition, which leads in high yield - without the need of sophisticated purification steps after the reaction - to a cyclic compound composition having a purity of 99% by weight or more and thus being pure enough to be used in a ring-opening polymerization method.

In accordance with the present invention this object is satisfied by providing a method for preparing a purified cyclic compound composition, wherein the method comprises the steps of:
i) providing in a reactor a reaction mixture containing at least one cyclizable linear compound and reacting the at least one cyclizable linear compound in a ring-closing reaction so as to obtain a crude composition containing at least one cyclic compound,
ii) feeding at least a portion of the crude composition obtained in step i) to a precipitator, in which the crude composition is cooled to a precipitation temperature being below the solubility temperature of at least one of the at least one cyclic compound contained in the crude composition for precipitating at least a portion thereof so as to obtain precipitated solid cyclic compound and unprecipitated liquid,
iii) separating at least a portion of the precipitated solid cyclic compound obtained in step ii) from the unprecipitated liquid so as to obtain a solid cyclic compound fraction and unprecipitated liquid fraction, and
iv) washing the solid cyclic compound fraction obtained in step iii) with a washing solvent having a temperature of above the precipitation temperature used in step ii) so as to obtain a washing solvent fraction and as purified cyclic compound composition a washed solid cyclic compound fraction.

This solution bases on the surprising finding that a method with the aforementioned steps and in particular comprising the step of washing the solid cyclic compound fraction with a solvent having a temperature of above the precipitation temperature used in precipitation step leads in a simple and fast manner as well as in a high yield to a cyclic compound composition being pure enough to be used in a ring-opening polymerization method for producing the respective polyester and more specifically having a purity of 99% by weight or more. The method in accordance with the present invention combines a selective precipitation of the target cyclic compound(s) and a washing of the precipitated target cyclic compound(s) with a solvent having a temperature of above the precipitation temperature used in the precipitation step. While the selective precipitation of the target cyclic compound(s) exploits the different melting point or solubility, respectively, of linear and cyclic compounds, the washing step efficiently purifies the target cyclic compound(s) to a particular high purity degree. It has been surprisingly found by the inventors of the present invention that the impurities formed during the ring-closing reaction and being trapped in the precipitate obtained in the precipitation step have a faster kinetic of re-dissolution in the solvent than the target cyclic compound(s), which allows to efficiently purify the target cyclic compound(s) in the washing step to the particular high purity degree of 99% by weight or even more. On account of the high purity, the target cyclic compound(s) may be directly used in a ring opening polymerization to produce respective polyesters and preferably aromatic polyesters without need of further yield-reducing purification steps, such as silica adsorption, column chromatography and/or distillation.

The term precipitation means in accordance with the present invention any kind of forming solids from a solution, independent from whether the solid is formed as amorphous solid or as crystal. Thus, the term precipitation includes any kind of crystallization forming crystals as well as any kind of other precipitation forming amorphous solid.

In accordance with a particular preferred embodiment of the present invention, the method further comprises a step of recycling the unprecipitated liquid fraction obtained in step iii) into the reactor of step i). The recycling of the unprecipitated liquid fraction obtained in step iii) into the reactor of step i) shifts the thermodynamic equilibrium of the reaction towards the formation of the at least one cyclic compound, which in turn shortens the reaction time and increases the yield.

In principle, the present invention is not particularly limited concerning the chemical nature of the at least one cyclizable linear compound being contained in the reaction mixture of step i). Cyclizable means in this connection that the cyclizable linear compound is able to undergo during a ring-closing reaction a cyclization to a cyclic compound. Preferably, in step i) a reaction mixture is provided, which contains at least one cyclizable linear compound comprising a first terminal group being a carboxylic acid group, an ester group or an acid halogenide group and a second terminal group being a hydroxy group, a carboxylic acid group, an ester group or an acid halogenide group.

In a further development of the idea of the present invention it is suggested that the at least one cyclizable linear compound comprises at least one aromatic hydrocarbon group, preferably at least one furan group and more preferably at least one furanoate group.

Good results are in particular obtained, when the both aforementioned embodiments are combined, i.e. if the at least one cyclizable linear compound contained in the reaction mixture comprises i) at least one aromatic hydrocarbon group, which is preferably at least one furan group and more preferably at least one furanoate group, further comprises ii) a first terminal group being a carboxylic acid group, an ester group or an acid halogenide group and further comprises iii) a second terminal group being a hydroxy group, a carboxylic acid group, an ester group or an acid halogenide group.

In a further particular preferred embodiment of the present invention, in step i) a reaction mixture is provided, which contains at least one cyclizable linear compound being
a) a compound according to the general formula (I): 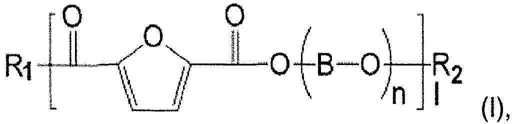 wherein:
   - R₁: is OH, OR, a halogen or O-B-OH,
   - R: is an optionally substituted linear, branched or cyclic alkyl, phenyl, aryl or alkylaryl,
   - B: is an optionally-substituted linear, branched or cyclic alkyl, phenyl, aryl or alkylaryl,
   - n: is an integer of 0 to 20,
   - I: is an integer of 1 to 100 and
   - R2: is H or 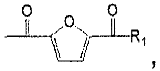
   and/or
b) a mixture of a compound according to the general formula (II) and of a compound according to the general formula (III): 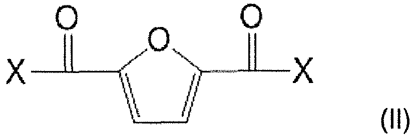 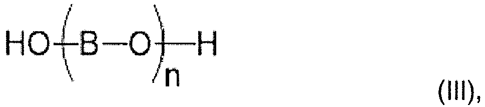 wherein:
   - B and n: are defined as above and
   - X: is an OH, a halogen or an optionally-substituted alkyloxy, phenoxy or aryloxy,

   wherein the compound according to the general formula (I) and/or the mixture of a compound according to the general formula (II) and of a compound according to the general formula (III) is reacted in step i) to a cyclic compound according to the general formula (IV): 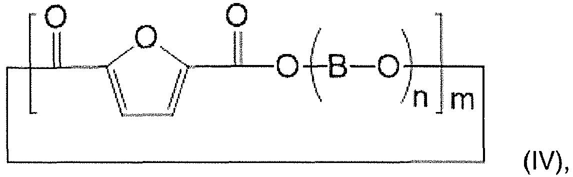
   wherein B and n are defined as above and m is an integer of 1 to 20.

Preferably, the reaction mixture provided in step i) comprises a compound according to the general formula (I) and/or a mixture of a compound according to the general formula (II) and of a compound according to the general formula (III), wherein n is an integer of 1 to 20, wherein m is an integer of 2 to 15, and wherein R₁, R, B, I, R₂ and X are defined as defined above. Particularly preferably, the reaction mixture provided in step i) comprises a respective compound according to the general formula (I).

Even more preferably, the reaction mixture provided in step i) comprises a compound according to the general formula (I) and/or a mixture of a compound according to the general formula (II) and of a compound according to the general formula (III), wherein n is an integer of 1 or of 2 to 10, wherein m is an integer of 3 to 10, and wherein R₁, R, B, I, R₂ and X are defined as defined above. Particularly preferably, the reaction mixture provided in step i) comprises a respective compound according to the general formula (I).

In a further development of the idea of the present invention it is suggested that the reaction mixture provided in step i) comprises a compound according to the general formula (I) and/or a mixture of a compound according to the general formula (II) and of a compound according to the general formula (III), wherein B in general formula (I) and/or in general formula (III) is an optionally-substituted linear, branched or cyclic alkyl or phenyl and preferably an optionally-substituted linear, branched or cyclic alkyl, and wherein R₁, R, n, I, R₂ and X are defined as defined above. It is also in this embodiment preferred that in the compound according to the general formula (I) and/or mixture of a compound according to the general formula (II) and of a compound according to the general formula (III), n is an integer of 1 to 20, m is an integer of 2 to 15 and R₁, R, B, I, R₂ and X are defined as defined above. More preferably, n is an integer of 1 or of 2 to 10, m is an integer of 3 to 10 and R₁, R, B, I, R₂ and X are defined as defined above. In a further preferred variant of this embodiment, B in general formula (I) and/or in general formula (III) is an optionally-substituted linear, branched or cyclic C₁₋₆-alkyl or phenyl and preferably an optionally-substituted linear, branched or cyclic C₁₋₆-alkyl, wherein R₁, R, n, I, R₂ and X are defined as defined above. Particular good results are obtained in this embodiment, when B in general formula (I) and/or in general formula (III) is -CH₂-CH₂-, wherein n is 1, wherein R₁ is OH, OR or O-CH₂-CH₂-OH, and wherein R, I, R₂ and X are defined as defined above. Particularly preferably, the reaction mixture provided in step i) comprises a respective compound according to the general formula (I).

The present invention is not particularly limited concerning the reaction conditions in step i). However, good results are in particular obtained, when the reaction mixture provided in step i) comprises a compound according to the general formula (I) and when the reaction mixture is reacted in step i) at a temperature of 100 to 350°C for 30 to 600 minutes. More preferably, the reaction mixture is reacted in step i) at a temperature of 150 to 300°C for 40 to 400 minutes and even more preferably at a temperature of 180 to 280°C for 50 to 300 minutes. The pressure is preferably between 0.01 and 1.5 MPa, more preferably between 0.05 and 0.5 MPa, yet more preferably between 0.075 and 0.15 MPa and most preferably ambient pressure, i.e. 1.0 MPa.

When the reaction mixture provided in step i) comprises a mixture of a compound according to the general formula (II) and of a compound according to the general formula (III), the reaction mixture is preferably reacted in step i) at a temperature of -10 to 150°C for 5 to 240 minutes and more preferably at a temperature of -5 to 100°C for 10 to 180 minutes. The pressure is preferably between 0.01 and 0.5 MPa, more preferably between 0.05 and 0,2 MPa, yet more preferably between 0.075 and 0,15 MPa and most preferably ambient pressure, i.e. 1.0 MPa.

In order to accelerate the reaction, the reaction mixture provided in step i) may further comprise a compound being selected from the group consisting of bases, catalysts, solvents and arbitrary combinations of two or more of such compounds. Preferably, the reaction mixture provided in step i) further comprises a base and a catalyst and even more preferably a base, a catalyst and a solvent.

Suitable examples for a base are compounds having the general formula (IV): 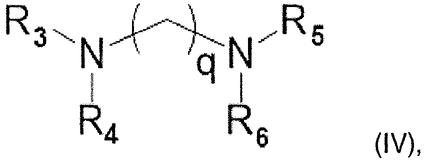 wherein each of the groups R₃ to R₆ are hydrogen, optionally-substituted alkyl, phenyl, aryl, or alkaryl, wherein each of the groups R₃ to R₆ may optionally be bonded together by a single or double bond group as part of a cyclic substituent in a cyclic optional organic base. More preferably, the reaction mixture provided in step i) comprises as base 1,4-diazabicyclo[2.2.2]octane (DABCO) or 1,8-diazabicyclo[5.4.0]undec-7-ene (DBU).

Another suitable example for a base is triazabicyclodecene (TBD).

In accordance with a further preferred embodiment of the present invention, the reaction mixture provided in step i) further comprises a catalyst. Preferably, the catalyst is a metal oxide alkoxide, a metal alkoxide or a metal carboxylate. Good results are in particular obtained, when the catalyst is a metal oxide alkoxide, a metal alkoxide or metal carboxylate, wherein the metal is selected from the group consisting of tin, zinc, titanium, aluminium and arbitrary combinations of two or more of these metals. Most preferably, the catalyst is a tin oxide alkoxide, such as in particular dibutyltin oxide.

In accordance with a yet further preferred embodiment of the present invention, the reaction mixture provided in step i) further comprises a solvent. Suitable solvents are aromatic solvents, preferably solvents being benzene, a substituted benzene, naphthalene or a substituted naphthalene and more preferably a solvent being selected from the group consisting of chlorobenzene, dichlorobenzene [such as o-dichlorobenzene], alkyl benzene [such as toluene, o-xylene, m-xylene or p-xylene], naphthalene and arbitrary combinations of two or more of the aforementioned solvents. Likewise, non-aromatic solvents may be used, such as preferably those being selected from the group consisting of alkylamides, dialkylsulfoxides, dialkylcarboxylates, tetrahydrofuran and arbitrary combinations of two or more of the aforementioned solvents and more preferably a solvent being selected from the group consisting of dimethylformamide, dimethylsulfoxide, dimethylcarbonate, diethylcarbonate, tetrahydrofuran and arbitrary combinations of two or more of the aforementioned solvents.

In order to increase the yield of the reaction, it is suggested in a further development of the idea of the present invention that the reaction in step i) is performed in a reactor, wherein during the reaction by-product(s) (e.g. diol byproduct(s), such as ethylene glycol) and optional a portion of the solvent is/are removed by evaporation and preferably distillation. For example, the ring-closing reaction in step i) may be performed in a single reaction vessel and the evaporated by-product(s) and optional portion of solvent is/are collected via condensation in a typically separate vessel.

Most preferably, the reaction in step i) is performed distillation assisted and most preferably as distillation assisted cyclodepolymerization (DA-CDP).

In step ii) of the method in accordance with the present invention, at least a portion (and preferably all) of the crude composition obtained in step i) is fed into a precipitator, in which the crude composition is cooled to a precipitation temperature being below the solubility temperature of at least one of the at least one cyclic compound contained in the crude composition for precipitating at least a portion thereof so as to obtain precipitated solid cyclic compound and unprecipitated liquid. Solubility temperature means in accordance with the present patent application the temperature at which the solubility saturation of at least one of at least one cyclic compound is reached so that, when the temperature is lowered below the solubility temperature, the respective cyclic compound starts to precipitate. Good results are in particular obtained, when in step ii) at least a portion (and preferably all) of the crude composition obtained in step i) is cooled in the precipitator to a precipitation temperature being 10 to 80°C below the solubility temperature of at least one of the cyclic compound being included in the crude composition, in order to obtain both, a fast, but also nearly complete precipitation of the target cyclic compound(s). More preferably, in step ii) at least a portion (and preferably all) of the crude composition obtained in step i) is cooled in the precipitator to a precipitation temperature being 10 to 50°C and most preferably 10 to 20°C below the solubility temperature of at least one of the cyclic compounds being included in the crude composition.

The preferred absolute precipitation temperature depends on the chemical nature of the at least one cyclic compound to be precipitated and purified with the method, i.e. of the at least one target cyclic compound. For instance, in step ii) at least a portion (and preferably all) of the crude composition obtained in step i) is fed into the precipitator and cooled therein to a precipitation temperature of 50 to 250°C, more preferably of 50 to 200°C and most preferably of 50 to 180°C. The aforementioned precipitation temperatures are in particular preferred, when the reaction mixture provided in step i) contains at least one cyclizable linear compound being a compound according to the general formula (I) and/or a mixture of a compound according to the general formula (II) and of a compound according to the general formula (III).

The present invention is not particularly limited concerning the kind of precipitation, respectively. Thus, the precipitation may be performed in step ii) statically or dynamically. For instance, the precipitation may be performed in a static precipitator, such as in a vessel or pipe, in which a plurality of cooled plates is arranged. Preferably, the precipitation is performed dynamically and more preferably as suspension precipitation, such as in a stirred vessel or pipe, in which the walls are cooled.

In a further development of the idea of the present invention, it is proposed that the mean residence time of the crude composition within the precipitator in step ii) is 15 to 120 minutes, preferably 15 to 90 minutes and more preferably 15 to 60 minutes.

In accordance with a further particular preferred embodiment of the present invention, the reaction step i) and the precipitation step ii) are performed continuously, wherein preferably a portion of the crude composition obtained in step i) is continuously withdrawn from the reactor and continuously fed into the precipitator of step ii), and wherein during step ii) continuously a portion of a mixture of precipitated solid cyclic compound and of unprecipitated liquid is withdrawn from the precipitator and fed into step iii).

After the precipitation, at least a portion and preferably all of the precipitated solid cyclic compound obtained in step ii) is separated from the unprecipitated liquid (which is usually denoted as "mother liquor") so as to obtain a solid cyclic compound fraction and unprecipitated liquid fraction. If the precipitation is performed in step ii) as static precipitation, in step iii) first the unprecipitated liquid is withdrawn from the precipitator. The remaining precipitates being deposited on the cooled surfaces of the static precipitator are then optionally subjected to one or more sweating steps, in order to increase the purity of the precipitates. Sweating means that the precipitates being deposited on the cooled surfaces of the static precipitator are gently heated to a temperature close to the melting temperature of cyclic compound in order to partially melt the precipitates. Trapped and adherent melt, which contains the impurities, drains off during the partial melting of the precipitates and is then removed from the precipitator. Finally, the (remaining) precipitates are molten and withdrawn from the static precipitator as solid cyclic compound fraction.

In the preferred embodiment, in which step ii) is performed dynamically and more preferably as suspension precipitation in step iii) a mixture of precipitated solid cyclic compound and of unprecipitated liquid is withdrawn from the precipitator and is fed into a solid-liquid-separator, in which the precipitated solid cyclic compound is separated from the unprecipitated liquid so as to obtain a solid cyclic compound fraction and unprecipitated liquid fraction. More preferably, in step iii) a mixture of precipitated solid cyclic compound and of unprecipitated liquid is continuously withdrawn from the precipitator and is continuously fed into a solid-liquid-separator, in which the precipitated solid cyclic compound is continuously separated from the unprecipitated liquid so as to obtain a solid cyclic compound fraction and unprecipitated liquid fraction. This embodiment is advantageously combined with the embodiment according to which the steps i) and ii) are performed continuously, so that the reaction step i), the precipitation step ii) and the separation step iii) are all performed continuously, namely wherein a portion of the crude composition obtained in step i) is continuously withdrawn from the reactor and continuously fed into the precipitator of step ii), wherein during step ii) continuously a portion of a mixture of precipitated solid cyclic compound and of unprecipitated liquid is withdrawn from the precipitator and fed into step iii) and wherein during step iii) continuously the precipitated solid cyclic compound is separated from the unprecipitated liquid so as to obtain a solid cyclic compound fraction and unprecipitated liquid fraction.

In a further development of the idea of the present invention it is suggested that the separation in step iii) is performed in one or more solid-liquid-separation devices being selected from the group consisting of centrifugal separators, filtration units, decanters and arbitrary combinations of two or more of these separation devices.

In accordance with one variant of this embodiment of the present invention, the separation in step iii) is performed in one or more centrifugal separators. The one or more centrifugal separators may be centrifuges, i.e. batchwise-operating separators. Preferably, the separation in step iii) is performed in a continuously operating centrifugal separator, such as in a cyclone separator and preferably in a tangential cyclone separator.

In accordance with another variant of this embodiment of the present invention, the separation in step iii) is performed in one or more filtration units. The one or more filtration units may be batchwise-operating filtration units. Preferably, the separation in step iii) is performed in a continuously operating filtration unit.

The present invention is not particularly limited concerning the chemical nature of the washing solvent used in step iv) as long as it is able to dissolve unprecipitated liquid being entrapped in the precipitate and as long as it does not significantly dissolve the solid cyclic compound of the precipitate. Preferably, the solvent used in step iv) is the solvent used in the step i), if in step i) a solvent is used. Thus, it is preferred that the washing solvent used in step iv) is selected from the group consisting of aromatic solvents non-aromatic washing solvents. Preferred aromatic washing solvents are benzene, a substituted benzene, naphthalene or a substituted naphthalene and more preferably a washing solvent being selected from the group consisting of chlorobenzene, dichlorobenzene [such as o-dichlorobenzene], alkyl benzene [such as toluene, o-xylene, m-xylene or p-xylene], naphthalene and arbitrary combinations of two or more of the aforementioned washing solvents. Preferred non-aromatic washing solvents are those being selected from the group consisting of alkylamides, dialkylsulfoxides, dialkylcarboxylates, tetrahydrofuran and arbitrary combinations of two or more of the aforementioned washing solvents and more preferably those being selected from the group consisting of dimethylformamide, dimethylsulfoxide, dimethylcarbonate, diethylcarbonate, tetrahydrofuran and arbitrary combinations of two or more of the aforementioned washing solvents. The aforementioned washing solvents are in particular preferred, when the reaction mixture provided in step i) contains at least one cyclizable linear compound being a compound according to the general formula (I) and/or a mixture of a compound according to the general formula (II) and of a compound according to the general formula (III).

Good results are in particular obtained, when the washing solvent used in step iv) has a temperature of 10 to 100°C, preferably of 10 to 80°C and more preferably of 10 to 60°C above the precipitation temperature used in step ii). For instance, during the washing step iv) a 5- to 1-fold and more preferably 1-fold volume of washing solvent based on the volume of the solid cyclic compound fraction being washed is used.

The preferred absolute temperature of the washing solvent depends on the chemical nature of the at least one cyclic compound to be washed in step iv), i.e. of the at least one target cyclic compound. For instance, the washing solvent used in step iv) has a temperature of 120 to 300Y°C, preferably of 120 to 250°C and more preferably of 120 to 200°C. The aforementioned washing solvent temperatures are in particular preferred, when the reaction mixture provided in step i) contains at least one cyclizable linear compound being a compound according to the general formula (I) and/or a mixture of a compound according to the general formula (II) and of a compound according to the general formula (III).

The washing in step iv) is preferably performed at a pressure of 0.01 to 1.5 MPa, more preferably of 0.05 to 0.5 MPa, yet more preferably of 0.075 to 0.15 MPa and most preferably at ambient pressure, i.e. 1.0 MPa.

The washing of the solid cyclic compound fraction in step iv) may performed in any suitable device, such as in a device being selected from the group consisting of stirred vessels, stirred columns, filtration units and arbitrary combinations of two or more of these devices. In addition, a spray washing vessel or a jet washing vessel may be used, in which the washing solvent is distributed during the operation onto the solid as droplets

If the washing of the solid cyclic compound fraction in step iv) is performed in a stirred vessel and/or in a stirred column, a mixture of washing solvent and of washed solid cyclic compound fraction is withdrawn from the stirred vessel and/or in a stirred column. This mixture is then preferably separated in a solid-liquid separation into a washed solid cyclic compound fraction and into a washing solvent fraction. The solid-liquid separation may be performed in one or more solid-liquid-separation devices being selected from the group consisting of centrifugal separators, filtration units, decanters and arbitrary combinations of two or more of these separation devices.

In a further particular preferred embodiment of the present invention, steps iii) and iv) are performed - simultaneously or subsequently - in one device. This embodiment is preferably performed in one or more filtration units. The one or more filtration units may work batchwise, i.e. firstly a mixture of precipitated solid cyclic compound and of unprecipitated liquid is fed into the one or more filtration units, in which secondly the mixture is filtrated so as to obtain a filter cake of precipitated solid cyclic compound, before thirdly the unprecipitated liquid is separated from the filter cake, whereupon fourthly washing solvent with an appropriate temperature is fed onto the filter cake of precipitated solid cyclic compound to thereby wash the filter cake, before fifthly the washing solvent is removed by filtration from the filter cake of precipitated solid cyclic compound.

Alternatively, the one or more filtration units may work semi-continuously. This may be realized for instance by performing steps iii) and iv) in two or more filtration units, wherein each of the two or more filtration units periodically performs a separation step, a washing step and a removal step of precipitated solid, wherein at least two of the filtration units perform at a given time a different step from the group of separation step, washing step and removal step, and wherein successively a portion of the mixture of precipitated solid cyclic compound and of unprecipitated liquid obtained in step ii) is fed into the respective filtration unit before starting the separation step, whereas washing solvent is fed into the respective filtration unit before starting the washing step and optionally also during the washing step and washing solvent passing the filter is withdrawn during the washing step.

For instance, steps iii) and iv) are performed in three filtration units, wherein each of the two or more filtration units periodically performs a separation step, a washing step and a removal step of precipitated solid. At a given time, each of the three filtration units performs a different step, namely a first of the three filtration units performs the separation step of separating precipitated solid cyclic compound obtained in step ii) from the unprecipitated liquid by filtration so as to obtain a filter cake of solid cyclic compound, whereas a second of the three filtration units performs the washing step of washing the filter cake of solid cyclic compound fraction with a washing solvent having a temperature of above the precipitation temperature used in step ii) so as to obtain a washing solvent fraction and a washed solid cyclic compound fraction, and whereas a third of the three filtration units performs the removal step of removing precipitated solid. A portion of the mixture of precipitated solid cyclic compound and of unprecipitated liquid obtained in step ii) is fed into the respective filtration unit before starting the separation step, whereas washing solvent is fed into the respective filtration unit before starting the washing step and optionally also during the washing step and washing solvent passing the filter is withdrawn during the washing step.

If the washing step iv) is performed for instance in a filtration unit so that the washed solid cyclic compound fraction does not contain anymore any washing solvent or, if at all, only traces thereof, the washed solid cyclic compound fraction as purified cyclic compound composition may then be used for instance for the production of polyester by a ring-opening polymerization without further purification. However, if the washed solid cyclic compound fraction contains after step iv) a small amount of washing solvent, it is preferred the that washed solid cyclic compound fraction is further purified in a step v) by separating remaining washing solution from the washed solid cyclic compound fraction. This separation is preferably performed by drying the washed solid cyclic compound fraction, if only a comparable small amount of washing solvent is contained in the washed solid cyclic compound fraction, for instance by drying the washed solid cyclic compound fraction at a temperature of 120 to 250°C and preferably at a temperature of 120 to 200°C.

If the washed solid cyclic compound fraction, however, contains still comparable high amounts of washing solvent, such as after performing the washing step in a vessel by mixing the solid cyclic compound fraction obtained in step iii) with the washing solvent, it is preferred to perform after step iv) as step v) a solid-liquid separation. Preferably, the solid-liquid separation is performed, as in the preferred embodiment of step iii), in one or more solid-liquid-separation devices being selected from the group consisting of centrifugal separators, filtration units, decanters and arbitrary combinations of two or more of these separation devices.

In order to increase the efficiency of the method and to minimize the waste, it is proposed in a further development of the idea of the present invention that the washing solution fraction obtained in step iv) is recycled as solvent into step iv). Preferably, the washing solution fraction obtained in step iv) is first purified, before it is then recycled as solvent into step iv). For instance, the purification may be performed by distillation, in order to separate the washing solution from dissolved or dispersed impurities.

If the washed solid cyclic compound fraction obtained in step iv) is dried, as described as an embodiment above, the fraction having been evaporated during the drying is preferably (also) recycled as solvent into step iv) and is more preferably first purified (for instance by distillation), before it is then recycled as washing solvent into step iv).

As set out above, the method in accordance with the present invention leads, in particular due to the washing step iv), to a highly pure cyclic compound composition having a purity of 99% by weight or more. As a particular advantage in comparison to the prior art methods, the method in accordance with the present invention does not need any further purification steps than those described above. Thus, it is particularly preferred that the method in accordance with the present invention does not comprise any purification step selected from the group consisting of silica adsorption, column chromatography, distillation and arbitrary combinations of two or more of these purification steps.

In accordance with a further aspect, the present invention relates to a purified cyclic compound composition being obtainable with the aforementioned method.

It is preferred that the purified cyclic compound composition has a purity of 99% by weight or more, more preferably of 99.5% by weight or more and most preferably of 99.9% by weight or more.

In accordance with another aspect, the present invention is related to the use of the above described purified cyclic compound composition, for example for preparing a polyester polymer composition comprising the step of reacting the purified cyclic compound composition in a ring-opening polymerization step.

Preferably, the ring-opening polymerization step is performed in the presence of a catalyst and under conditions of a reaction temperature and a reaction time being sufficient to yield a polyester polymer. Preferably, the polyester polymer is derived from a compound according to the general formula (I) and has a weight average molecular weight of 10,000 to 80,000 g/mol and more preferably of 10,000 to 60,000 g/mol.

In yet a further aspect, the present invention is related to a plant for preparing a purified cyclic compound composition, wherein the plant comprises:
a) a reactor comprising one or more inlets and one or more outlets,
b) a precipitator comprising an inlet being connected with an outlet of the reactor and comprising one or more outlets,
c) a device comprising an inlet being connected with at least one of the one or more outlets of the precipitator and comprising one or more outlets, wherein the device c) is selected from the group consisting of centrifugal separators, filtration units, decanters and arbitrary combinations of two or more of these separation devices.

In a further development of the idea of the present invention, it is proposed that the plant further comprises a recycle line connecting an outlet of device c) and an inlet of the reactor i).

In accordance with a preferred embodiment of the present invention, the plant further comprises d) a device being selected from the group consisting of stirred vessels, stirred columns, filtration units and arbitrary combinations of two or more of these devices, wherein the device d) comprises an inlet being connected with at least one of the one or more outlets of the device c) and comprises one or more outlets. It is preferred that the device d) of this embodiment is a stirred vessel and/or a stirred column.

Moreover, it is preferred that in the aforementioned embodiment the plant comprises as device c) a centrifugal separator and as device d) a stirred vessel or stirred column, wherein the device c) comprises an outlet for liquid and an outlet for solid, wherein the outlet for solid is connected with an inlet of device d).,The device c) further comprises an inlet for washing solvent, wherein the outlet of device d) is connected with the inlet of a solid-liquid separator e) being selected from the group consisting of centrifugal separators, filtration units, decanters and arbitrary combinations of two or more of these separation devices, wherein the solid-liquid separator e) comprises an outlet for liquid and an outlet for solid, and wherein preferably the outlet for liquid of device c) is connected with an inlet of the reactor i).

In accordance with an alternative preferred embodiment of the present invention, the device c) of this plant comprises one or more filtration units. Preferably, the device c) of this embodiment comprises three filtration units. Each of the three filtration units is connected with an inlet line for washing solvent as well with an inlet line for crude composition from the reactor, with an outlet line for washing solvent, with an outlet line for washed solid cyclic compound and with a recycle line leading to the reactor. The three outlet lines for washing solvent combine to a central line for washing solvent and the three outlet lines for washed solid cyclic compound combine to a central line for washed solid cyclic compound. All three filtration units are arranged in parallel and each of the three filtration units periodically performs a separation step, a washing step and a removal step of precipitated solid. Each of the filtration units performs at a given time a different step from the group of separation step, washing step and removal step, namely one at the a given time performs the separation step, while another one performs the washing step and the third one performs the removal step. During the separation step, the inlet line for crude composition and the recycle line of the filtration unit are open, whereas the inlet line for washing solvent, the outlet line for washing solvent and the outlet line for precipitated solid are closed, so that mixture being withdrawn from the precipitator is fed into the respective filtration unit and pushed through the filter of the respective filtration unit to collect the precipitate in the filter as filter cake, whereas the remaining unprecipitated liquid is led back via the recycle line of the respective filtration unit into the reactor. During the washing step, the inlet line for washing solvent and the outlet line for washing solvent are open, whereas the line for crude composition, the recycle line and the outlet line for precipitated solid of the respective filtration unit are closed, so that washing solvent is fed into the respective filtration unit and pushed through the filter of the respective filtration unit wash the filter cake, whereas the washing solvent is withdrawn via the outlet line of the respective filtration unit and optionally led into a separator 48, where the washing solvent is separated from impurities being dissolved and/or dispersed therein. During the removal step, the line for crude composition, the inlet line for washing solvent, the outlet line for washing solvent and the recycle line are closed, whereas the line of the respective filtration unit is open, so that precipitated solid is withdrawn from the respective filtration unit and optionally led into the drying unit 40, where the solid is dried.

Subsequently, the present invention is described by means of illustrative, but not limiting figures, in which:
- Fig. 1: shows schematically a plant for performing the method in accordance with one embodiment of the present invention.
- Fig. 2: shows schematically a plant for performing the method in accordance with another embodiment of the present invention.

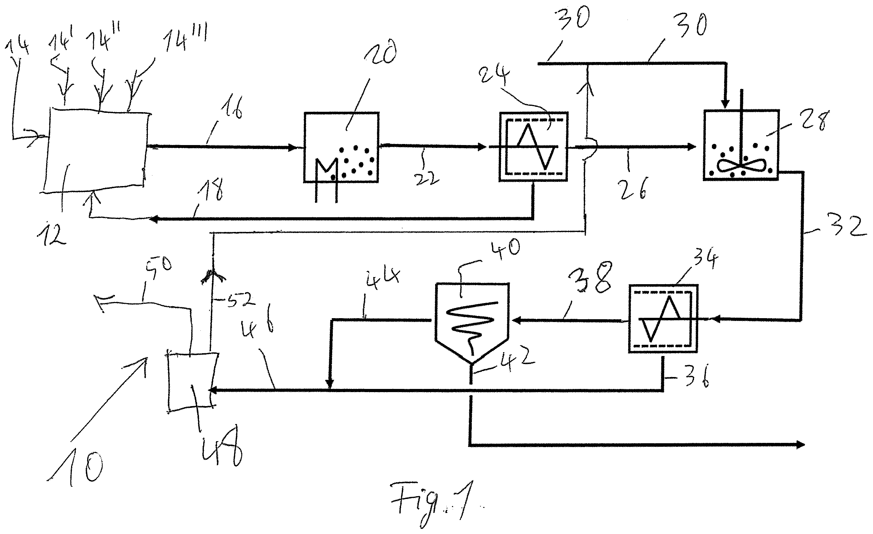

The plant 10 shown in figure 1 comprises a reactor 12, which comprises an inlet line 14 for cyclizable linear compound, an inlet line 14' for base, an inlet line 14" for catalyst, an inlet line 14‴ for solvent and an outlet line 16 for crude composition containing at least one cyclic compound. Moreover, a recycle line 18 leads into the reactor 12. Moreover, the plant 10 comprises a precipitator 20, which comprises an inlet being connected with the outlet line 16 of the reactor 12 and which comprises an outlet line 22 for a mixture of precipitated solid cyclic compound and unprecipitated liquid. In addition, the plant 10 comprises a centrifuge 24, which comprises an inlet being connected with the outlet line 22 of the precipitator 20, an outlet for unprecipitated liquid being connected with the recycle line 18 and an outlet line 26 for solid cyclic compound fraction. Downstream of the centrifuge 24, a washing tank 28 in form of a stirred vessel is arranged, which comprises an inlet line 30 for washing solvent and which comprises an outlet line 32 for a mixture of washing solvent and of washed solid cyclic compound. The outlet line 32 of the washing tank 28 is connected with an inlet of a further centrifuge 34, which further comprises an outlet line 36 for liquid and an outlet line 38 for solid, wherein the outlet line 38 for solid of the centrifuge 34 is connected with the inlet of a drying unit 40. The drying unit 40 comprises an outlet 42 for solid and an outlet 44 for liquid, wherein the outlet lines 42 and 44 are combined to a liquid line 46, which leads into a separator 48 for separating solvent from impurities being dissolved and/or dispersed therein. The separator 48 comprises an outlet line 50 for impurities and a recycle line 52 for washing solvent, which leads into the inlet line 30 of the washing tank 28.

During the operation of the plant 10, a cyclizable linear compound, such as one in accordance with the general formula (I), a base, a catalyst and solvent are fed via inlet lines 14, 14', 14", 14‴ into the reactor 12 and form therein a reaction mixture, which is reacted in a ring-closing reaction to a crude composition containing at least one cyclic compound. A portion of the crude composition is withdrawn from the reactor 12 and fed via line 16 into the precipitator 20, in which the crude composition is cooled to a precipitation temperature being below the solubility temperature of at least one of the at least one cyclic compound contained in the crude composition for precipitating at least a portion thereof so as to obtain a mixture of precipitated solid cyclic compound and unprecipitated liquid. This mixture is withdrawn from the precipitator 20 and led via the outlet line 22 into the centrifuge 24, in which the mixture is separated into an unprecipitated liquid fraction and into a solid cyclic compound fraction. While the unprecipitated liquid fraction is withdrawn via line 18 from the centrifuge 20 and is recycled via line 18 into the reactor 12, the solid cyclic compound fraction is withdrawn from the centrifuge 20 via outlet line 26 and led into the washing tank 28. In addition to the solid cyclic compound fraction, washing solvent having a temperature of above the precipitation temperature used in the precipitator 20 is fed via line 30 into the washing tank 28. The washing tank 28 is a stirred vessel, in which the washing solvent and the solid cyclic compound fraction are mixed so as to wash the solid cyclic compound fraction with the washing solvent. The so obtained mixture of solid cyclic compound fraction and of washing solvent is led via line 32 into the centrifuge 34, in which the mixture is separated into a washing solvent fraction and into a washed solid cyclic compound fraction. While the washing solvent fraction is withdrawn from the centrifuge 34 via line 36 and is purified in the separator 48 by removing impurities therefrom, whereupon the purified washing solvent is recycled via lines 52, 30 into the washing tank 28, the washed solid cyclic compound fraction is fed via line 38 into the drying unit 40, in which remaining liquid is separated from the solid cyclic compound fraction. While the separated liquid is led via lines 44, 46 into the separator 48, the dried solid cyclic compound is withdrawn as the purified cyclic compound composition via line 42 from the plant 10.

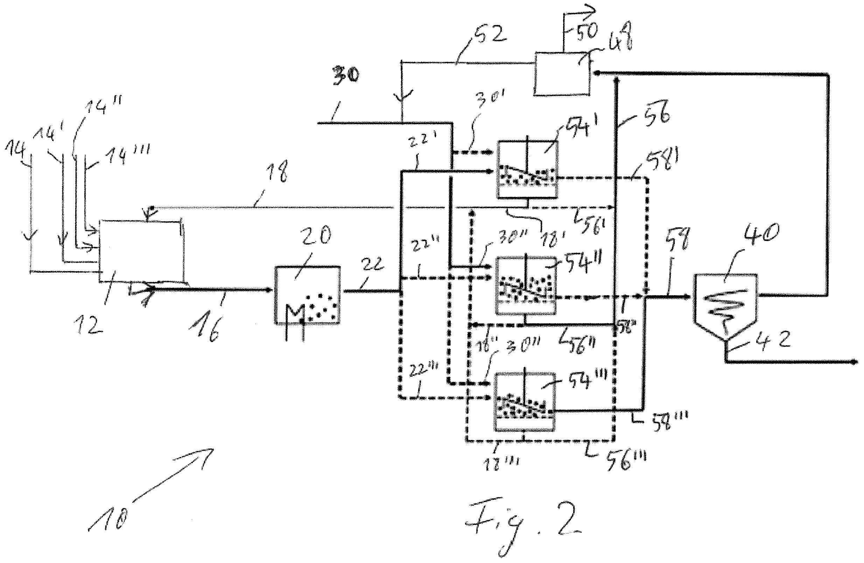

The plant 10 shown in figure 2 differs from that shown in figure 1 in that the plant 10 does not comprise two centrifuges 24, 34 and a washing tank, but instead thereof three filtration units 54', 54", 54‴. Each of the three filtration units is connected with an inlet line 30', 30", 30‴ for washing solvent as well with an inlet line 22', 22", 22‴ for crude composition from the reactor, with an outlet line 56', 56", 56‴ for washing solvent, with an outlet line 58', 58", 58‴ for washed solid cyclic compound and with a recycle line 18', 18", 18‴ leading to the reactor 12. The three outlet lines 56', 56", 56‴ combine to a central line 56 and the three outlet lines 58', 58", 58‴ combine to a central line 58. All three filtration units 54', 54", 54‴ are arranged in parallel and each of the three filtration units 54', 54", 54‴ periodically performs a separation step, a washing step and a removal step of precipitated solid. Each of the filtration units 54', 54", 54‴ performs at a given time a different step from the group of separation step, washing step and removal step, namely at the time shown in figure 2 the filtration unit 54' performs the separation step, the filtration unit 54" performs the washing step and the filtration unit 54‴ performs the removal step. During the separation step, the line 22' and the recycle line 18' of the filtration unit 54' are open (indicated by the unbroken line), whereas the inlet line 30' for washing solvent, the outlet line 56' for washing solvent and the outlet line 58' for precipitated solid are closed (indicated by the broken line), so that mixture being withdrawn from the precipitator 20 is fed into the respective filtration unit 54' and pushed through the filter of the respective filtration unit 54' to collect the precipitate in the filter as filter cake, whereas the remaining unprecipitated liquid is led back via the recycle line 18' of the respective filtration unit 54' into the reactor 12. During the washing step, the inlet line 30" for washing solvent and the outlet line 56" for washing solvent are open (indicated by the unbroken line), whereas the line 22", the recycle line 18" and the outlet line 58" for precipitated solid of the respective filtration unit 54" are closed (indicated by the broken line), so that washing solvent is fed into the respective filtration unit 54" and pushed through the filter of the respective filtration unit 54" to wash the filter cake, whereas the washing solvent is withdrawn via the outlet line 56" of the respective filtration unit 54" and led into the separator 48, where it is processed as described above for figure 1. During the removal step, the line 22‴, the inlet line 30‴ for washing solvent, the outlet line 56‴ for washing solvent and the recycle line 18‴ are closed (indicated by the broken line), whereas the line 58‴ of the respective filtration unit 54‴ is open (indicated by the unbroken line), so that precipitated solid is withdrawn from the respective filtration unit 54‴ and led into the drying unit 40, where it is processed as described above for figure 1.

### Reference Numerals

- 10: Plant
- 12: Reactor
- 14: Inlet line for cyclizable linear compound of reactor
- 14': Inlet line for base of reactor
- 14": Inlet line for catalyst of reactor
- 14‴: Inlet line for solvent of reactor
- 16: Outlet line of reactor
- 18, 18', 18", 18‴: Recycle line
- 20: Precipitator
- 22, 22', 22", 22‴: Outlet line of precipitator
- 24: Centrifuge
- 26: Outlet line of centrifuge
- 28: Washing tank
- 30, 30', 30", 30‴: Inlet line of washing tank
- 32: Outlet line of washing tank
- 34: Centrifuge
- 36: Outlet line for liquid of centrifuge
- 38: Outlet line for solid of centrifuge
- 40: Drying unit
- 42: Outlet for purified cyclic compound composition of the drying unit
- 44: Outlet for liquid of the drying unit
- 46: Liquid line
- 48: Separator
- 50: Outlet line for impurities
- 52: Recycle line for washing solvent
- 54', 54", 54‴: Filtration units
- 56, 56', 56", 56‴: Outlet line for washing solvent
- 58, 58', 58", 58‴: Outlet line for solid

## Claims

1. A method for preparing a purified cyclic compound composition, wherein the method comprises the steps of:
i) providing in a reactor a reaction mixture containing at least one cyclizable linear compound and reacting the at least one cyclizable linear compound in a ring-closing reaction so as to obtain a crude composition containing at least one cyclic compound,
ii) feeding at least a portion of the crude composition obtained in step i) to a precipitator, in which the crude composition is cooled to a precipitation temperature being below the solubility temperature of at least one of the at least one cyclic compound contained in the crude composition for precipitating at least a portion thereof so as to obtain precipitated solid cyclic compound and unprecipitated liquid,
iii) separating at least a portion of the precipitated solid cyclic compound obtained in step ii) from the unprecipitated liquid so as to obtain a solid cyclic compound fraction and unprecipitated liquid fraction, and
iv) washing the solid cyclic compound fraction obtained in step iii) with a washing solvent having a temperature of above the precipitation temperature used in step ii) so as to obtain a washing solvent fraction and as purified cyclic compound composition a washed solid cyclic compound fraction.

2. The method of claim 1, wherein the method further comprises a step of recycling the unprecipitated liquid fraction obtained in step iii) into the reactor of step i).

3. The method of claim 1 or 2, wherein in step i) a reaction mixture is provided, which contains at least one cyclizable linear compound being
a) a compound according to the general formula (I) 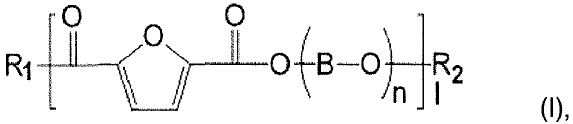 wherein:
R₁ is OH, OR, a halogen or O-B-OH,
R is an optionally substituted linear, branched or cyclic alkyl, phenyl, aryl or alkylaryl,
B is an optionally-substituted linear, branched or cyclic alkyl, phenyl, aryl or alkylaryl,
n is an integer of 0 to 20,
I is an integer of 1 to 100 and
R2 is H or 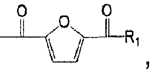
and/or
b) a mixture of a compound according to the general formula (II) and of a compound according to the general formula (III) 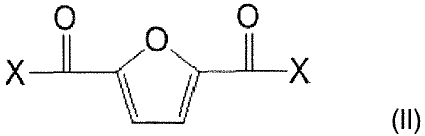 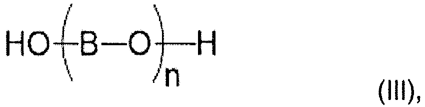 wherein:
B and n are defined as above and
X is an OH, a halogen or an optionally-substituted alkyloxy, phenoxy or aryloxy,
wherein the compound according to the general formula (I) and/or the mixture of a compound according to the general formula (II) and of a compound according to the general formula (III) is reacted in step i) to a cyclic compound according to the general formula (IV): 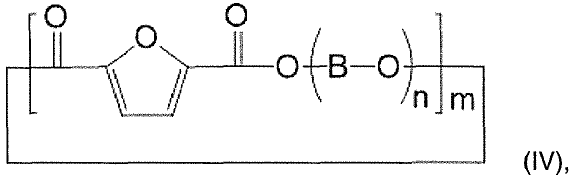
wherein B and n are defined as above and m is an integer of 1 to 20, wherein preferably the reaction mixture provided in step i) comprises a compound according to the general formula (I) and/or a mixture of a compound according to the general formula (II) and of a compound according to the general formula (III), wherein B in general formula (I) and/or in general formula (III) is -CH₂-CH₂-CH₂-CH₂-, wherein n is 1, wherein R₁ is OH, OR or O-CH₂-CH₂-CH₂-CH₂-OH, and wherein R, I, R₂ and X are defined as defined in claim 5.

4. The method of any of the preceding claims, wherein the reaction mixture provided in step i) further comprises a solvent, wherein the solvent is selected from the group consisting of benzene, substituted benzenes, naphthalene, substituted naphthalenes, alkylamides, dialkylsulfoxides, dialkylcarboxylates, tetrahydrofuran and arbitrary combinations of two or more of the aforementioned solvents, and more preferably is selected from the group consisting of chlorobenzene, dichlorobenzene, alkyl benzene, naphthalene dimethylformamide, dimethylsulfoxide, dimethylcarbonate, diethylcarbonate, tetrahydrofuran and arbitrary combinations of two or more of the aforementioned washing solvents.

5. The method of any of the preceding claims, wherein in step ii) at least a portion of the crude composition obtained in step i) is fed into the precipitator and cooled therein to a precipitation temperature being 10 to 80°C, preferably 10 to 50°C and more preferably 10 to 20°C below the solubility temperature of at least one of the cyclic compound being included in the crude composition, and/or wherein in step ii) at least a portion of the crude composition obtained in step i) is fed into the precipitator and cooled therein to a precipitation temperature of 50 to 250°C, preferably of 50 to 200°C and more preferably of 50 to 180°C.

6. The method of any of the preceding claims, wherein steps i) and ii) are performed continuously, wherein a portion of the crude composition obtained in step i) is continuously withdrawn from the reactor and continuously fed into the precipitator of step ii), and wherein during step ii) continuously a portion of a mixture of precipitated solid cyclic compound and of unprecipitated liquid is withdrawn from the precipitator and fed into step iii).

7. The method of any of the preceding claims, wherein a) the separation in step iii) is performed in a centrifuge and preferably in a continuously operating centrifugal separator, preferably a tangential cyclone separator, and/or wherein the separation in step iii) is performed in one or more filtration units and preferably in a continuously operating filtration unit.

8. The method of any of the preceding claims, wherein the washing solvent used in step iv) is selected from the group consisting of benzene, substituted benzenes, naphthalene, substituted naphthalenes, alkylamides, dialkylsulfoxides, dialkylcarboxylates, tetrahydrofuran and arbitrary combinations of two or more of the aforementioned washing solvents, and more preferably is selected from the group consisting of chlorobenzene, dichlorobenzene, alkyl benzene, naphthalene dimethylformamide, dimethylsulfoxide, dimethylcarbonate, diethylcarbonate, tetrahydrofuran and arbitrary combinations of two or more of the aforementioned washing solvents.

9. The method of any of the preceding claims, wherein the washing solvent used in step iv) has a temperature of 10 to 100°C, preferably of 10 to 80°C and more preferably of 10 to 60°C above the precipitation temperature used in step ii), and/or wherein the washing solvent used in step iv) has a temperature of 120 to 300°C, preferably of 120 to 250°C and more preferably of 120 to 200°C.

10. The method of any of the preceding claims, wherein steps iii) and iv) are performed in two or more filtration units, wherein each of the two or more filtration units periodically performs a separation step, a washing step and a removal step of washing solvent, wherein at least two of the filtration units perform at a given time a different step from the group of separation step, washing step and removal step, and wherein successively a portion of the mixture of precipitated solid cyclic compound and of unprecipitated liquid obtained in step ii) is fed into the respective filtration unit before starting the separation step, whereas washing solvent is fed into the respective filtration unit before starting the washing step and optionally also during the washing step.

11. The method of any of the preceding claims, which does not comprise any purification step selected from the group consisting of silica adsorption, column chromatography, distillation and arbitrary combinations of two or more of these purification steps.

12. A purified cyclic compound composition being obtainable with a method of any of the preceding claims.

13. Plant for preparing a purified cyclic compound composition, wherein the plant comprises:
a) a reactor comprising one or more inlets and one or more outlets,
b) a precipitator comprising an inlet being connected with an outlet of the reactor and comprising one or more outlets,
c) a device comprising an inlet being connected with at least one of the one or more outlets of the precipitator and comprising one or more outlets, wherein the device c) is selected from the group consisting of centrifugal separators, filtration units, decanters and arbitrary combinations of two or more of these separation devices.

14. The plant of claim 13, which further comprises a recycle line connecting an outlet of device c) and an inlet of the reactor i).

15. The plant of claim 13 or 14, which further comprises d) a device being selected from the group consisting of stirred vessels, stirred columns, filtration units and arbitrary combinations of two or more of these devices, wherein the device d) comprises an inlet being connected with at least one of the one or more outlets of the device c) and comprises one or more outlets, wherein the plant comprises as device c) a centrifugal separator and as device d) a stirred vessel or stirred column, wherein the device c) comprises an outlet for liquid and an outlet for solid, wherein the outlet for solid is connected with an inlet of device d), wherein the device c) further comprises an inlet for washing solvent, wherein the outlet of device d) is connected with the inlet of a solid-liquid separator e) being selected from the group consisting of centrifugal separators, filtration units, decanters and arbitrary combinations of two or more of these separation devices, wherein the solid-liquid separator e) comprises an outlet for liquid and an outlet for solid, and wherein preferably the outlet for liquid of device c) is connected with an inlet of the reactor i).
